# EUROPEAN PATENT APPLICATION

(11) **EP 3 586 724 A1**
(43) Date of publication of application: **01.01.2020**
(21) Application number: 18180110.1
(22) Date of filing: 27.06.2018
(51) Int. Cl.: A61B 5/00, A61B 5/053

(54) **DEVICE FOR USE IN DETERMINING A HYDRATION LEVEL OF SKIN**

(71) Applicant: Koninklijke Philips N.V., 5656 AE Eindhoven (NL)
(72) Inventor: VARGHESE, Babu, 5656 AE Eindhoven (NL); SPOORENDONK, Wouter, 5656 AE Eindhoven (NL); VAN DEN AKER, Karel, 5656 AE Eindhoven (NL)
(74) Representative: de Haan, Poul Erik

(57) **Abstract**

There is provided a device (100) for use in determining a hydration level of skin (200). The device (100) comprises at least two electrodes (102) configured to provide an electrical signal to the skin (200) and a spacer (104) arranged such that the at least two electrodes (102) provide the electrical signal to the skin (200) at different penetration depths (106, 108). The device (100) also comprises a detector (110) configured to measure a response received from the skin (200) at the different penetration depths (106, 108) for use in determining the hydration level of the skin (200).

## Description

### FIELD OF THE INVENTION

The disclosure relates to a device and a method of operating the device for use in determining a hydration level of skin.

### BACKGROUND OF THE INVENTION

A hydration (e.g. moisture or water) level of skin or, more specifically, the stratum corneum, is considered an important factor in determining skin appearance and skin health. The most well-established commercially available existing devices for determining a hydration level of skin (e.g. the Corneometer® CM 820, the Skicon® 200, and the Nova DPM® 9003) measure dielectric properties of skin, such as permittivity and conductivity. However, it is difficult for these devices to measure the hydration level of the skin accurately, since the measurements acquired by the devices are dependent on the amount of superficial lipids, such as sebum or oil, and also other factors, such as the presence of sweat, the presence of hairs, the presence of artificial oil (e.g. moisturizing creams), the surface microtopography, and environmental factors (e.g. humidity and temperature).

Thus, it is expected that the measurement of a hydration level of skin obtained with existing devices can be affected by other factors. For example, lipids can have a very low value of dielectric constant compared to that of water. As such, if a thin layer of lipids is present in the probing depth of an existing device (which is typically in the range of few tens of microns), the hydration level of skin determined using such a device will naturally be impacted when the determination is based on the relatively high dielectric constant of water. Although a thin layer of lipids does not change the absolute baseline hydration level of skin directly, the skin hydration level measurements obtained by the existing devices are influenced due to the difference in the dielectric properties of lipids and water.

US 2008/0045816 discloses an apparatus for simultaneously measuring skin moisture content and a sweat glad activity to provide information to the user on both of these measurements. However, the skin moisture content measured by this apparatus is still affected by the presence of sweat, lipids and other factors. As such, this apparatus is also not capable of providing accurate measurements of the hydration level of skin.

### SUMMARY OF THE INVENTION

As noted above, a limitation with existing devices is that they provide inaccurate measurements of the hydration level of skin due to the dependence of a hydration level measurement on the amount of lipids present on the surface of the skin. It would thus be valuable to have an improvement to address the existing problems.

Therefore, according to a first aspect, there is provided a device for use in determining a hydration level of skin. The device comprises at least two electrodes configured to provide an electrical signal to the skin and a spacer arranged such that the at least two electrodes provide the electrical signal to the skin at different penetration depths. The device also comprises a detector configured to measure a response received from the skin at the different penetration depths for use in determining the hydration level of the skin.

In some embodiments, the penetration depths may comprise any one or more of a penetration depth from an electrode of the at least two electrodes to a surface of the skin and a penetration depth from an electrode of the at least two electrodes to a location beneath the surface of the skin.

In some embodiments, the spacer may comprise a dielectric spacer. In some embodiments, the spacer may be patterned such that the at least two electrodes provide electrical signal to the skin at the different penetration depths. In some embodiments, a composition and/or thickness of the spacer may be selected based on a geometry of the at least two electrodes.

In some embodiments, the spacer may comprise a polyethylene terephthalate material, a polyelectric material, an aluminium material, or a polyetheretherketone material. In some embodiments, the spacer may be of a predefined thickness in a range from 1 micron to 100 microns. In some embodiments, the spacer may be arranged such that the spacer is positioned between one or more of the at least two electrodes and the skin in use.

In some embodiments, the at least two electrodes may comprise at least two pixelated electrodes.

In some embodiments, the device may further comprise a signal generator configured to generate the electrical signal and provide the electrical signal to the at least two electrodes.

According to a second aspect, there is provided a system for determining a hydration level of skin. The system comprises a device as described earlier and a processor. The processor is configured to acquire, from the detector, the measured response received from the skin at the different penetration depths and determine the hydration level of the skin based on the measured response received from the skin at the different penetration depths.

According to a third aspect, there is provided a method of operating a device for use in determining a hydration level of skin. The device comprises at least two electrodes, a spacer, and a detector. The method comprises providing, by the at least two electrodes, an electrical signal to the skin. The spacer is arranged such that the at least two electrodes provide the electrical signal to the skin at different penetration depths. The method also comprises measuring, by the detector, a response received from the skin at the different penetration depths for use in determining the hydration level of the skin.

In some embodiments, the method may further comprise generating, by a signal generator, the electrical signal and providing, by the signal generator, the electrical signal to the at least two electrodes

In some embodiments, the method may further comprise acquiring, by a processor from the detector, the measured response received from the skin at the different penetration depths and determining, by the processor, the hydration level of the skin based on the measured response received from the skin at the different penetration depths.

According to a fourth aspect, there is provided a computer program product comprising a computer readable medium, the computer readable medium having a computer readable code embodied therein, the computer readable code being configured such that, on execution by a suitable computer or processor, the computer or processor is caused to perform the method described above.

According to the aspects and embodiments described above, the limitations of existing techniques are addressed. In particular, according to the above-described aspects and embodiments, it is possible to determine a hydration level of skin more accurately. This is possible since the confounding influence of lipids on the hydration level measurements is minimized by the spacer of the device enabling the skin response to be measured at different penetration depths for use in determining the hydration level of the skin. There is thus provided an improved device and method of operating the device for determining a hydration level of skin, which is aimed at overcoming existing problems.

These and other aspects will be apparent from and elucidated with reference to the embodiment(s) described hereinafter.

### BRIEF DESCRIPTION OF THE DRAWINGS

Exemplary embodiments will now be described, by way of example only, with reference to the following drawings, in which:
Fig. 1 is a block diagram of a device according to an embodiment;
Fig. 2 illustrates example spacers according to embodiments;
Fig. 3 is flow chart illustrating a method according to an embodiment;
Fig. 4 illustrates skin hydration measurements obtained by an existing device;
Fig. 5 illustrates skin hydration measurements obtained by an existing device;
Fig. 6 illustrates skin hydration measurements obtained using a skin response measured by a device according to an embodiment;
Fig. 7 illustrates skin hydration measurements obtained using a skin response measured by a device according to another embodiment; and
Fig. 8 illustrates a comparison of skin hydration measurements obtained using a skin response measured by a device according to an embodiment and an existing device.

### DETAILED DESCRIPTION OF EMBODIMENTS

As noted above, there is provided herein an improved device and method of operating the device for determining a hydration (e.g. moisture or water) level of skin. In some embodiments, the device described herein can be a device for treating skin and/or for diagnosing one more skin conditions. In other embodiments, the device described herein can be a separate (e.g. a stand-alone) device for use with a device for treating skin and/or for diagnosing one more skin conditions. The device described herein can be for determining a hydration level of skin from any part of the body of a subject, such as a finger (e.g. the device may be a finger print device), a thumb, a face, or any other part of the body of the subject.

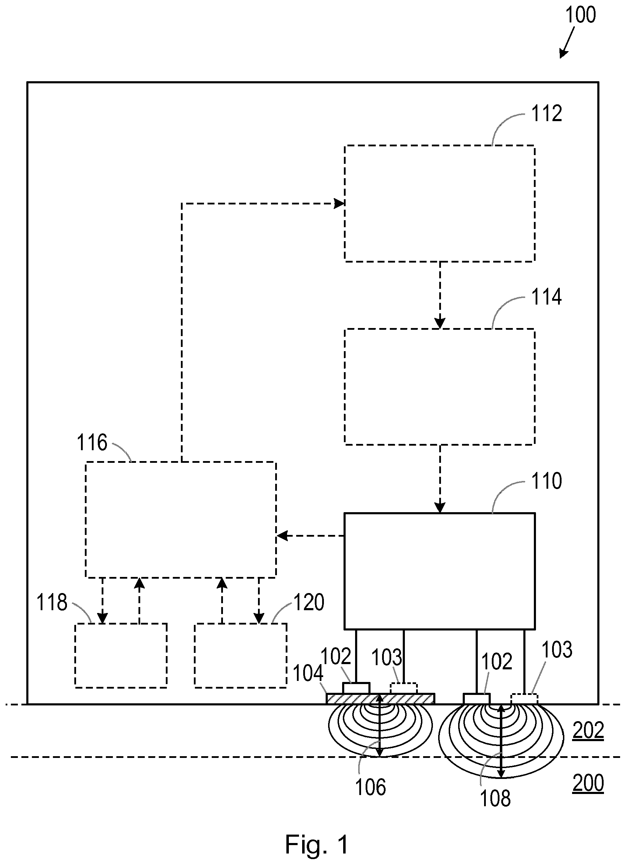

Fig. 1 illustrates a device 100 for use in determining a hydration level of skin 200. As illustrated in Fig. 1, the device 100 comprises at least two electrodes 102 configured to provide an electrical signal to the skin 200. As illustrated in Fig. 1, the device 100 may also comprise at least two further electrodes 103, each further electrode configured to receive the electrical signal provided to the skin 200 by one of the at least two electrodes 102. In some embodiments, the at least two electrodes 102 may comprise at least two active electrodes 102 configured to transmit the electrical signal and the at least two further electrodes 103 may comprise at least two return electrodes 103 configured to receive the electrical signal transmitted from a corresponding one of the at least two active electrodes 102. Thus, in some embodiments, the device 100 can comprise at least two pairs of electrodes or an array of electrodes. The at least two pairs of electrodes or the array of electrodes can comprise the at least two active electrodes 102 and at least two corresponding return electrodes 103.

In embodiments where the device 100 comprises at least two active electrodes 102 and at least two return electrodes 103, the electrical signal can be provided across each active electrode 102 and corresponding return electrode 103. In use, each active electrode 102, corresponding return electrode 103 and a lipid layer 202 on the skin 200 (if present) or the skin 200 and the lipid layer 202 on the skin 200 (if present) form an electrical circuit. Thus, an electrical signal provided across an active electrode 102 and a corresponding return electrode 103 passes through a lipid layer 202 on the skin 200 (if present) or the skin 200 and the lipid layer 202 on the skin 200 (if present) between the active electrode 102 and corresponding return electrode 103. As illustrated in Fig. 1, the electrical signal can comprise a plurality of electrical field lines between each active electrode 102 and corresponding return electrode 103.

In some embodiments, any one or more of the at least two active electrodes 102 and the at least two return electrodes 103 can comprise a microelectrode. An active electrode of the at least two active electrodes 102 may be located at any suitable distance from a return electrode of the at least two return electrodes 103. For example, in some embodiments, an active electrode of the at least two active electrodes 102 may be located at a distance in a range from 0.1 mm to 1 mm from a return electrode of the at least two return electrodes 103. In this way, the sensitivity of the device 100 may be increased. In some embodiments, the at least two electrodes 102 configured to provide an electrical signal to the skin 200 may comprise at least two pixelated electrodes.

A person skilled in the art will be familiar with electrodes that are suitable for providing an electrical signal to skin 200 and also electrodes that are suitable for receiving an electrical signal from the skin 200. A person skilled in the art will also be familiar with an arrangement and/or a geometry for the at least two active electrodes 102 that is suitable for providing an electrical signal to skin 200 and also for the at least two return electrodes 103 that is suitable for receiving an electrical signal from the skin 200. For example, the at least two active electrodes 102 and/or the at least two return electrodes 103 may be provided in an interlaced arrangement, a multi-pin arrangement, a concentric arrangement, a skin chip arrangement, or any other arrangement of which the skilled person will be aware.

As illustrated in Fig. 1, the device 100 also comprises a spacer (or a spacer material) 104. The spacer 104 is arranged such that the at least two electrodes 102 provide the electrical signal to the skin 200 at different penetration (or probing) depths 106, 108. In some embodiments, the penetration depths 106, 108 may comprise a penetration depth 106 that is confined to a lipid (e.g. sebum) layer 202 on the skin 200 (if present). For example, the penetration depths 106, 108 may comprise a penetration depth 106 that is from an electrode of the at least two electrodes 102 to a surface of the skin 200. In these embodiments, as illustrated in Fig. 1, where the electrical signal comprises a plurality of electrical field lines between each active electrode 102 and corresponding return electrode 103, the electrical field lines can be confined to the lipid layer 202 on the skin 200 (if present). Alternatively, or in addition, in some embodiments, the penetration depths 106, 108 may comprise a penetration depth 108 that is from an electrode of the at least two electrodes 102 to a location beneath the surface of the skin 200. In these embodiments, as illustrated in Fig. 1, where the electrical signal comprises a plurality of electrical field lines between each active electrode 102 and corresponding return electrode 103, the electrical field lines are not confined to the lipid layer 202 on the skin 200 but also extend beneath the surface of the skin 200.

In some embodiments, the different penetration depths 106, 108 can comprise a depth 106 of up to 10 microns, for example up to 9 microns, for example up to 8 microns, for example up to 7 microns, for example up to 6 microns, for example up to 5 microns, for example up to 4 microns, for example up to 3 microns, for example up to 2 microns, for example up to 1 micron and a depth 108 of up to 40 microns, for example up to 35 microns, for example up to 30 microns, for example up to 25 microns, for example up to 20 microns.

In some embodiments, as illustrated in Fig. 1, the spacer 104 can be arranged such that the spacer 104 is positioned between one or more of the at least two electrodes 102 configured to provide an electrical signal to the skin 200 and the skin 200 in use. In some embodiments, the spacer 104 can be patterned (or segmented). The spacer 104 can be patterned such that the at least two electrodes provide the electrical signal to the skin 200 at the different penetration depths 106, 108. For example, the spacer 104 may be patterned such that, in use, at least part of the spacer 104 is positioned between one or more of the at least two electrodes 102 and the skin 200. In some embodiments, the spacer 104 maybe patterned such that, in use, at least part of the spacer 104 is positioned between alternate ones of the at least two electrodes 102 and the skin 200. In effect, one or more of the at least two electrodes 102 may be provided with a spacer 104, while the remainder of the at least two electrodes 102 may be provided without a spacer 104.

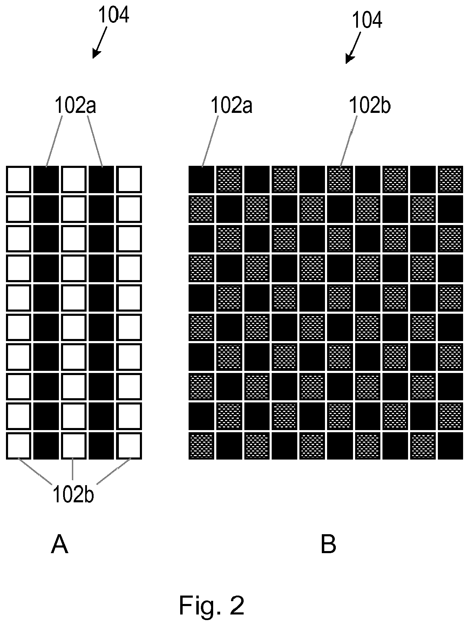

Fig. 2 illustrates example spacers 104 according to some embodiments, where the spacer 104 is patterned such that the at least two electrodes provide the electrical signal to the skin 200 at the different penetration depths 106, 108. In the embodiment illustrated in Fig. 2A, the spacer 104 is patterned such that, in use, at least part of the spacer 104 is positioned between the skin 200 and each alternate row of the at least two electrodes 102. That is, alternate rows of the at least two electrodes 102 may be provided with the spacer 104 according to some embodiments. Thus, in effect, in the embodiment illustrated in Fig. 2A, a plurality of the at least two electrodes 102 are provided with the spacer 104 and a plurality of the at least two electrodes are provided without the spacer 104. In Fig. 2A, the areas provided with the spacer 104 are illustrated by the black squares 102a and the areas provided without the spacer 104 are illustrated by the white squares 102b. In some embodiments, each of the areas 102a, 102b may comprise at least two active electrodes 102. According to some embodiments, the distance between these at least two active electrodes 102 and their size can define the penetration depth 106, 108, with the penetration depth 106 of the at least two electrodes 102 provided with the spacer 104 being confined to a shallower depth than the penetration depth 108 of the at least two electrodes provided without the spacer 104.

In the embodiment illustrated in Fig. 2B, the spacer 104 is patterned such that, in use, at least part of the spacer 104 is positioned between alternate ones of the at least two electrodes 102 and the skin 200. That is, alternate ones of the at least two electrodes 102 are provided with the spacer 104. Thus, in effect, in the embodiment illustrated in Fig. 2B, a plurality of the at least two electrodes 102 are provided with the spacer 104 and a plurality of the at least two electrodes 102 are provided without the spacer 104. In Fig. 2B, the areas provided with the spacer 104 are illustrated by the black squares 102a and the areas provided without the spacer 104 are illustrated by the grey squares 102b. In some embodiments, each of the areas 102a, 102b may comprise at least two active electrodes 102. According to some embodiments, the distance between these at least two active electrodes 102 and their size can define the penetration depth 106, 108, with the penetration depth 106 of the at least two electrodes 102 provided with the spacer 104 being confined to a shallower depth than the penetration depth 108 of the at least two electrodes provided without the spacer 104.

In some embodiments, the spacer 104 may comprise a material that is at least partially insulating and at least partially conducting, such that at least some electrical signals pass through the spacer 104 to the skin 200. In some embodiments, the spacer 104 may comprise a dielectric spacer. In some embodiments, the spacer 104 may comprise a polyethylene terephthalate (PET) material, a polyelectric (PZE) material, an aluminium material, or a polyetheretherketone (PEEK) material. In some embodiments, the spacer 104 may comprise a foil.

In some embodiments, a composition (or type) of the spacer 104 maybe selected based on a geometry of the at least two electrodes 102. Alternatively, or in addition, in some embodiments, a thickness of the spacer 104 may be selected based on the geometry of the at least two electrodes 102. For example, in some embodiments, the composition and/or thickness of the spacer 104 may be different for different electrode geometries. In embodiments where the composition and/or thickness of the spacer 104 is selected, the composition and/or thickness of the spacer 104 may define the different penetration depths 106, 108. In some embodiments, the spacer 104 may be of a predefined thickness. The predefined thickness may, for example, be of the order of few microns to tens of microns. For example, in some embodiments, the spacer 104 may be of a predefined thickness in a range from 1 micron to 100 microns.

Returning back to Fig. 1, as illustrated, the device 100 further comprises a detector 110. The detector 110 is configured to measure a response received from the skin 200 at the different penetration depths 106, 108 for use in determining the hydration level of the skin 200. In effect, the spacer 104 can enable the detector 110 to acquire dual measurements with a spacer 104 and without a spacer 104. The detector 110 may be configured to measure the response received from the skin 200 at the different penetration depths 106, 108 via at least two return electrodes 103.

In some embodiments, the detector 110 can be configured to measure the response received from the skin 200 at the different penetration depths 106, 108 by being configured to measure a voltage across each active electrode 102 and corresponding return electrode 103. Alternatively, or in addition, in some embodiments, the detector 110 can be configured to measure the response received from the skin 200 at the different penetration depths 106, 108 by being configured to measure a current along the circuit formed by each active electrode 102, corresponding return electrode 103 and a lipid layer 202 on the skin 200 (if present) or the skin 200 and the lipid layer 202 on the skin 200 (if present). Alternatively, or in addition, in some embodiments, the detector 110 can be configured to measure the response received from the skin 200 at the different penetration depths 106, 108 by being configured to measure an impedance of the circuit formed by each active electrode 102, corresponding return electrode 103 and a lipid layer 202 on the skin 200 (if present) or the skin 200 and the lipid layer 202 on the skin 200 (if present). In the latter case, the detector 110 may comprise an impedance measurement system.

As illustrated in Fig. 1, in some embodiments, the device 100 may comprise a signal generator 112. However, it will be understood that, in other embodiments, the signal generator 112 may be external to (i.e. separate to or remote from) the device 100. For example, in some embodiments, the signal generator 112 can be a separate entity or part of another device. The signal generator 112 can be configured to generate the electrical signal. The signal generator 112 can also be configured to provide the electrical signal to the at least two electrodes 102. For example, the signal generator 112 can be configured to provide the electrical signal across each active electrode 102 and corresponding return electrode 103.

In some embodiments, the signal generator 112 can be configured to generate the electrical signal at a certain (e.g. predefined) frequency. In some embodiments, the signal generator 112 may be configured to generate frequency pulses. The frequency pulses may, for example, be fixed frequency pulses or variable frequency pulses. In some embodiments, the pulses may comprise low-voltage pulses. In some embodiments, the electrical signal may comprise a radiofrequency (RF) signal. Thus, in some embodiments, the signal generator 112 can be configured to generate a radiofrequency (RF) signal. In some of these embodiments, where the signal generator 112 is configured to generate frequency pulses, the frequency pulses may comprise radiofrequency pulses.

As illustrated in Fig. 1, in some embodiments, the device 100 may comprise an amplifier 114, such as a radiofrequency (RF) amplifier. In these embodiments, the signal generator 112 can be configured to provide the electrical signal to the at least two electrodes 102 via the amplifier 114. The amplifier 114 can be configured to amplify the electrical signal provided by (or a voltage of an output of) the signal generator 112.

As illustrated in Fig. 1, in some embodiments, the device 100 may comprise a processor 116. However, it will be understood that, in other embodiments, the processor 116 may be external to (i.e. separate to or remote from) the device 100. For example, in some embodiments, the processor 116 can be a separate entity or part of another device. The processor 116 may also be referred to as a control system.

In some embodiments, the processor 116 may be configured to control the at least two electrodes 102 to provide the electrical signal to the skin 200. In some embodiments, the processor 116 may be configured to control the at least two electrodes 102 to control any one or more of a frequency, a voltage and a pulse duration of the electrical signal that the at least two electrodes 102 are configured to provide to the skin 200. In some embodiments, the processor 116 may be configured to control the detector 110 to measure the response received from the skin 200 at the different penetration depths 106, 108. In some embodiments, the processor 116 can be configured to acquire, from the detector 110, the measured response received from the skin 200 at the different penetration depths 106, 108. For example, the output of the detector 110 can be provided to (e.g. fed into) the processor 116 according to some embodiments.

In some embodiments, the processor 116 can be configured to determine the hydration level of the skin 200 based on the measured response received from the skin 200 at the different penetration depths 106, 108. For example, in some embodiments, the processor 116 may be configured to determine the hydration level of the skin 200 by being configured to process the measured response received from the skin 200 at the different penetration depths 106, 108 to determine a measure of a permittivity at the different penetration depths 106, 108 (ε1, ε2). Thus, in effect, the processor 116 can be configured to measure the permittivity with the spacer 104 (ε1) and without the spacer 104 (ε2). In these embodiments, the processor 116 can be configured to determine the hydration level of the skin 200 based on the measured response received from the skin 200 at the different penetration depths 106, 108 by way of a ratio of the measure of permittivity at the different penetration depths 106, 108 (ε1, ε2), e.g. the hydration level of the skin 200 may be determined as (ε2- ε1)/ (ε2* ε1).

In some embodiments, the processor 116 can be configured to apply an offset correction to correct for the contribution of the spacer 104 to the permittivity measured with the spacer 104 (ε1). This provides a net permittivity (ε3) excluding the offset correction. The offset correction can, for example, be defined based on the type and/or thickness of the spacer 104. In embodiments where an offset correction is applied, the processor 116 can be configured to determine the hydration level of the skin 200 based on the measured response received from the skin 200 at the different penetration depths 106, 108 by way of a ratio of the corrected measure of permittivity at the different penetration depths 106, 108 (ε1, ε3), e.g. the hydration level of the skin 200 may be determined as (ε3- ε1)/ (ε3* ε1).

A person skilled in the art will be aware of various methods by which a permittivity may be measured from a response received from the skin 200. Also, although an example has been provided for the way in which the processor 116 may be configured to determine the hydration level of the skin 200 based on the measured response received from the skin 200 at the different penetration depths 106, 108, it will be understood that alternative ways (e.g. alternative permittivity ratios) are also possible and a person skilled in the art will be aware of such alternative ways in which the processor 116 may be configured to determine the hydration level of the skin 200 based on the measured response received from the skin 200 at the different penetration depths 106, 108.

Generally, in embodiments comprising a processor 116, the processor 116 can be configured to control the operation of the device 100 to implement the method described herein. In some embodiments, the processor 116 may comprise one or more processors. The one or more processors can be implemented in numerous ways, with software and/or hardware, to perform the various functions described herein. In some embodiments, each of the one or more processors can be configured to perform individual or multiple steps of the method described herein. In particular implementations, the one or more processors can comprise a plurality of software and/or hardware modules, each configured to perform, or that are for performing, individual or multiple steps of the method described herein. The one or more processors may comprise one or more microprocessors, one or more multi-core processors and/or one or more digital signal processors (DSPs), one or more processing units, and/or one or more controllers (such as one or more microcontrollers) that may be configured or programmed (e.g. using software or computer program code) to perform the various functions described herein.

In some implementations, for example, the processor 116 may comprise a plurality of (for example, interoperated) processors, processing units and/or modules, multi-core processors and/or controllers configured for distributed processing. It will be appreciated that such processors, processing units and/or modules, multi-core processors and/or controllers may be located in different locations and may perform different steps and/or different parts of a single step of the method described herein. The one or more processors may be implemented as a combination of dedicated hardware (e.g. amplifiers, pre-amplifiers, analog-to-digital convertors (ADCs) and/or digital-to-analog convertors (DACs)) to perform some functions and one or more processors (e.g. one or more programmed microprocessors, DSPs and associated circuitry) to perform other functions.

As illustrated in Fig. 1, in some embodiments, the device 100 may comprise a memory 118. Alternatively, or in addition, the memory 118 may be external to (e.g. separate to or remote from) the device 100. The processor 116 maybe configured to communicate with and/or connect to the memory 118. The memory 118 may comprise any type of non-transitory machine-readable medium, such as cache or system memory including volatile and non-volatile computer memory such as random-access memory (RAM), static RAM (SRAM), dynamic RAM (DRAM), read-only memory (ROM), programmable ROM (PROM), erasable PROM (EPROM), and electrically erasable PROM (EEPROM). In some embodiments, the memory 118 can be configured to store program code that can be executed by the processor 116 to cause the device 100 to operate in the manner described herein. Alternatively, or in addition, in some embodiments, the memory 118 can be configured to store information required by or resulting from the method described herein. For example, in some embodiments, the memory 118 may be configured to store any one or more of the response received from the skin 200 at the different penetration depths 106, 108, a hydration level of the skin 200 that may be determined using the received response, or any other information, or any combination of information, required by or resulting from the method described herein. In some embodiments, the processor 116 can be configured to control the memory 118 to store information required by or resulting from the method described herein.

As illustrated in Fig. 1, in some embodiments, the device 100 may comprise a user interface 120. Alternatively, or in addition, the user interface 120 may be external to (e.g. separate to or remote from) the device 100. The processor 116 may be configured to communicate with and/or connect to a user interface 120. The user interface 120 can be configured to render (e.g. output, display, or provide) information required by or resulting from the method described herein. For example, in some embodiments, the user interface 120 may be configured to render (e.g. output, display, or provide) any one or more of the response received from the skin 200 at the different penetration depths 106, 108, a hydration level of the skin 200 that may be determined using the received response, or any other information or any other information, or any combination of information, required by or resulting from the method described herein. Alternatively, or in addition, the user interface 120 can be configured to receive a user input. For example, the user interface 120 may allow a user to manually enter information or instructions, interact with and/or control the device 100. Thus, the user interface 120 maybe any user interface that enables the rendering (or outputting, displaying, or providing) of information and, alternatively or in addition, enables a user to provide a user input.

For example, the user interface 120 may comprise one or more switches, one or more buttons, a keypad, a keyboard, a mouse, a touch screen or an application (e.g. on a smart device such as a tablet, a smartphone, smartwatch, or any other smart device), a display or display screen, a graphical user interface (GUI) such as a touch screen, or any other visual component, one or more speakers, one or more microphones or any other audio component, one or more lights (e.g. light emitting diode LED lights), a component for providing tactile or haptic feedback (e.g. a vibration function, or any other tactile feedback component), a smart device (e.g. a smart mirror, a tablet, a smart phone, a smart watch, or any other smart device), or any other user interface, or combination of user interfaces. In some embodiments, the user interface that is controlled to render information may be the same user interface as that which enables the user to provide a user input. In some embodiments, the processor 116 can be configured to control the user interface 120 to operate in the manner described herein.

Although not illustrated in Fig. 1, in some embodiments, the device 100 may comprise a communications interface (or communications circuitry). Alternatively, or in addition, the communications interface may be external to (e.g. separate to or remote from) the device 100. The communications interface can be for enabling the device 100, or components of the device 100, to communicate with and/or connect to one or more other components, sensors, interfaces, devices, or memories (such as any of those described herein). For example, the communications interface can be for enabling any one or more of the earlier described at least two (active) electrodes 102, at least two return electrodes 103, detector 110, signal generator 112, amplifier 114, processor 116, memory 118, and user interface 120 to communicate with and/or connect to each other. The communications interface may enable the device 100, or components of the device 100, to communicate and/or connect in any suitable way. For example, the communications interface may enable the device 100, or components of the device 100, to communicate and/or connect wirelessly, via a wired connection, or via any other communication (or data transfer) mechanism. In some wireless embodiments, for example, the communications interface may enable the device 100, or components of the device 100, to use radio frequency (RF), Bluetooth, or any other wireless communication technology to communicate and/or connect.

Although also not illustrated in Fig 1, the device 100 may comprise a battery or other power supply for powering the device 100 or means for connecting the device 100 to a mains power supply. It will be understood that Fig. 1 only shows the components required to illustrate an aspect and, in a practical implementation, the device 100 may comprise any other component to those described herein or any combination of components.

In addition to the device 100, there is also provided a system for determining a hydration level of skin 200. The system comprises the device 100 described earlier for use in determining a hydration level of skin 200. The system can also comprise any one or more of the earlier described at least two return electrodes 103, signal generator 112, amplifier 114, processor 116, memory 118, and user interface 120.

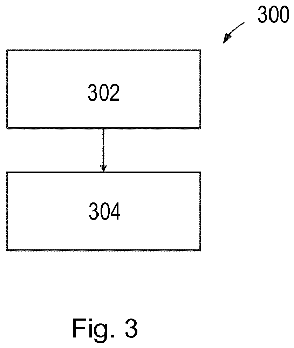

Fig. 3 illustrates a method 300 of operating the device 100 described earlier for use in determining a hydration level of skin 200. As described earlier, the device 100 comprises at least two electrodes 102, a spacer 104 and a detector 110. In some embodiments, the method 300 illustrated in Fig. 3 can be performed under the control of the processor 116 described earlier. At block 302 of Fig. 3, an electrical signal is provided to the skin 200. More specifically, the electrical signal is provided by the at least two electrodes 102. As described earlier, the spacer 104 is arranged such that the at least two electrodes 102 provide the electrical signal to the skin 200 at different penetration depths 106, 108. At block 304 of Fig. 3, a response received from the skin 200 at the different penetration depths 106, 108 for use in determining the hydration level of the skin 200 is measured. More specifically, the response is measured by the detector 110.

Although not illustrated in Fig. 3, in some embodiments, the method 300 may comprise generating the electrical signal and providing the electrical signal to the at least two electrodes 102. More specifically, the method 300 may comprise the electrical signal being generated and provided by the signal generator 112. Although also not illustrated in Fig. 3, in some embodiments, the method 300 may comprise acquiring the measured response received from the skin 200 at the different penetration depths 106, 108 and determining the hydration level of the skin 200 based on the measured response received from the skin 200 at the different penetration depths 106, 108. More specifically, the method 300 may comprise the measured response being acquired by the processor 116 from the detector 110 and the processor 116 determining the hydration level of the skin 200. In some embodiments, the method 300 may comprise relaying the determined hydration level of the skin 200 (e.g. when the determined hydration level of the skin 200 is outside a predefined range according to some embodiments) to the user interface 120. More specifically, the method 300 may comprise the determined hydration level of the skin 200 being relayed by the processor 116 to the user interface 120. It will be understood that the method 300 may comprise any other steps, and any combination of steps, corresponding to the operation of the device 100 described earlier with reference to Figs. 1 and 2.

As mentioned earlier, the device 100 and method 300 described herein enables a hydration level of skin 200 to be determined more accurately. This is possible since the confounding influence of lipids on the hydration level measurements is minimized by the spacer 104 of the device 100 enabling the skin 200 response to be measured at different penetration depths for use in determining the hydration level of the skin 200. Although a thin layer of lipids does not change the absolute baseline hydration level of skin directly, the skin hydration level measurements obtained by the existing devices are influenced due to the difference in the dielectric properties of lipids and water.

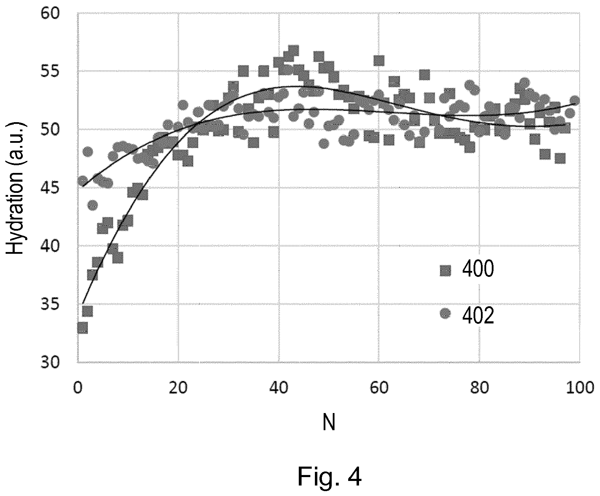

This can be observed in Fig. 4, which illustrates skin hydration measurements obtained from a Corneometer and normalized to the maximum value. The skin hydration measurements are based on skin capacitance and are obtained from the T-zone of forehead (illustrated by the points 400) and the forearm (illustrated by the points 402), which are known to have different levels of sebum content. In particular, the T-zone of forehead has a high level of sebum content, whereas the forearm has a low level of sebum content. In Fig. 4, the normalized skin hydration measurements (on the y-axis) are plotted against the number of measurements N taken at the same location (on the x-axis).

It can be seen from Fig. 4 that measurements repeated on the same location (N=100) showed that the hydration measurements gradually increase. This is due to the local occlusion when a probe of the device is in contact with the skin and due to the reduction in the amount of sebum after each measurement. In particular, each measurement removes some sebum, which means that successive measurements lead to a reduction in sebum and thus an increase in the hydration measurements. The increase is more pronounced on the forehead and, in particular, during the initial measurements when sebum is consistently present before it is gradually removed after each subsequent measurement. Thus, it can be seen from Fig. 4 that the existing methods used for skin hydration measurements can be influenced by the quantity of lipids on the surface of the skin.

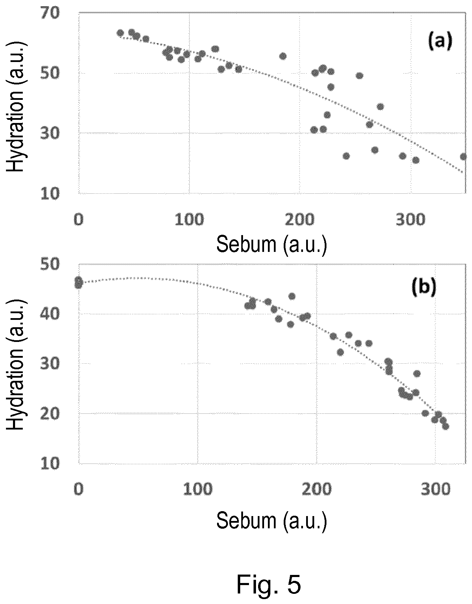

This can also be observed in Fig. 5, which illustrates a skin hydration level measured with a Corneometer normalized to the maximum value (on the y-axis) and plotted as a function of sebum level measured with a Sebumeter (on the x-axis). As can be seen from Fig. 5, the skin hydration measured with the Corneometer decreases as the sebum level increases, even though the absolute skin hydration is not changing.

In more detail, Fig. 5(a) shows an increase in hydration level as the use of the Sebumeter on the skin removes sebum for each measurement that is obtained. In this example, the hydration level is kept constant at a baseline level of 45 arbitrary units (a.u.) while the sebum is gradually removed. As illustrated in Fig. 5(a), the sebum levels drop from 300 arbitrary units (a.u.) to approximately 50 arbitrary units (a.u.) as the corresponding hydration levels increase from 20 to 60 arbitrary units (a.u.). Fig. 5(b) shows a decrease in hydration level as an amount of oil is increased by applying increasing amounts of artificial sebum on the skin. The hydration level decreases by a factor of two, from 40 to 20 arbitrary units (a.u.) when a thin layer of sebum is applied to the skin. In this example, the hydration level is kept constant at a baseline level of 45 arbitrary units (a.u.) while sebum concentration is increased by adding artificial sebum up to a final concentration of 1.2 µl/cm², which corresponds to a sebum level of 300 arbitrary units (a.u.).

Thus, as illustrated in Fig. 5, the hydration level depends strongly on the amount of lipids present at the surface of the skin. This insight into the dependency of the hydration level on the amount of lipids present at the surface of the skin has led to the device 100 and method 300 described herein, which overcomes the limitations associated with the dependency to enable a hydration level of skin 200 to be determined more accurately.

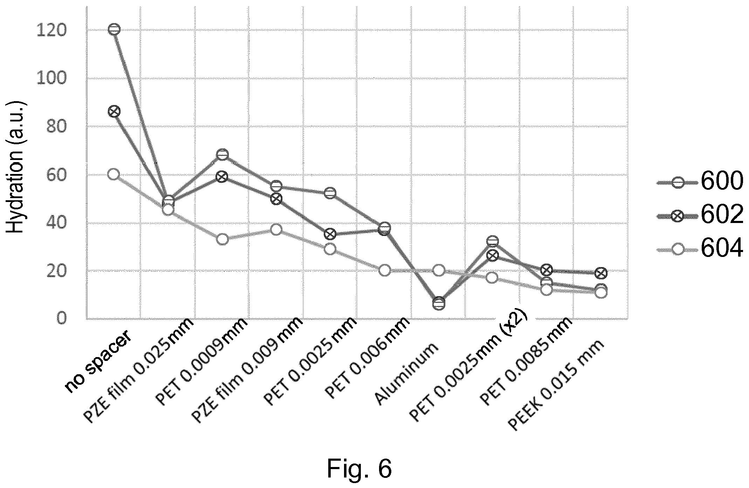

Fig. 6 illustrates the effect of using the device 100 described herein comprising different spacers 104 to measure a response received from the skin 200 at the different penetration depths 106, 108 for use in determining the hydration level of the skin 200. In more detail, Fig. 6 illustrates skin hydration measurements derived from the response measured using the device 100 and normalized to the maximum value (on the y-axis). The normalized skin hydration measurements are plotted as a function of material type and thickness for the spacer 104 (on the x-axis). For comparison purposes, Fig. 6 also illustrates skin hydration measurements obtained from an existing device with no spacer. The skin hydration measurements are provided in arbitrary units (a.u.) and the thickness is provided in millimeters (mm) in Fig. 6. The skin hydration measurements are obtained from a calibration pad soaked with liquids of different permittivity, namely Corneometer calibration (CK) solution 600, Ethyleen Glycol 602, and 1-Butanol 604.

A decrease in the skin hydration measurements can be seen in Fig. 6. This decrease is related to a decreasing penetration depth and the physical properties of the materials used for the spacer 104, such as the thickness and permittivity of the spacer 104. A factor related to the material used for the spacer 104 can be determined and compensated by a correction factor. If the observed decrease in the skin hydration measurements were only related to the presence of the spacer 104, the skin hydration measurements would not depend on the permittivity of the liquids in which the calibration pad is soaked as they do in Fig. 6. Thus, as illustrated in Fig. 6, it can be beneficial to confine the penetration depth 106 to superficial layers at or on the surface of the skin 200 by using the spacer 104 described herein. Furthermore, it can be seen from Fig. 6 that the confinement of the penetration depth 106 is different for different materials and thickness of spacer 104.

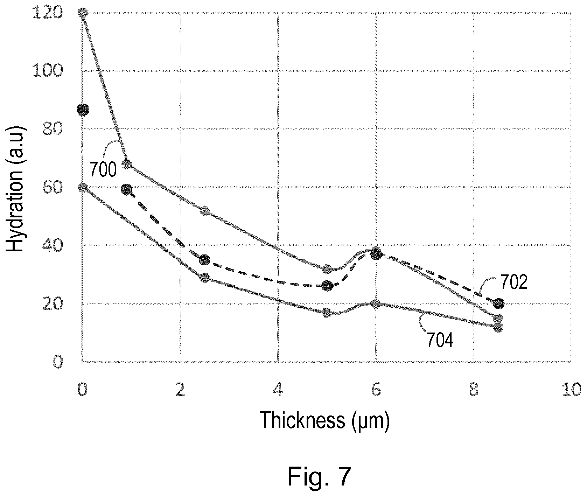

Fig. 7 illustrates the effect of using the device 100 comprising a spacer 104 as described herein to measure a response received from the skin 200 at the different penetration depths 106, 108 for use in determining the hydration level of the skin 200. In this illustrated example, the spacer 104 comprises a polyethylene terephthalate (PET) foil. In more detail, Fig. 7 illustrates skin hydration measurements derived from the response measured using the device 100 and normalized to the maximum value (on the y-axis). The normalized skin hydration measurements are plotted as a function of the thickness of the spacer 104 (on the x-axis). The skin hydration measurements are provided in arbitrary units (a.u.) and the thickness is provided in micrometers (µm) in Fig. 7. The skin hydration measurements are obtained from a calibration pad soaked with liquids of different permittivity, namely a Corneometer calibration (CK) solution 700, Ethyleen Glycol 702, and 1-Butanol 704.

As illustrated in Fig. 7, it can be beneficial to confine the penetration depth 106 to superficial layers at or on the surface of the skin 200 by using the spacer 104 described herein. Furthermore, it can be seen from Fig. 7 that the confinement of the penetration depth 106 depends on the thickness of spacer 104. It can be seen that, the thicker the spacer 104, the larger the confinement of the penetration depth using the spacer 104. For completeness, it is noted that some of the thickness measurements illustrated are a combination of two spacers 104 on top of each other. When this is the case (e.g. at 5 and 8.5 µm), air between the spacers 104 lowers the actual hydration level measurements compared to a single layer spacer (e.g. at 6 µm) where no air is present.

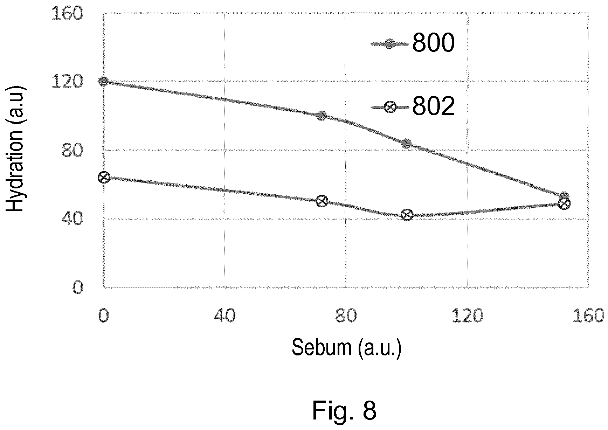

Fig. 8 illustrates a comparison of the influence of an amount (or level) of sebum on skin hydration measurements obtained using an existing device without a spacer (illustrated by the line labelled 800) and skin hydration measurements derived from the response measured using the device 100 comprising the spacer 104 described herein (illustrated by the line labelled 802). In more detail, Fig. 8 illustrates the skin hydration measurements normalized to the maximum value (on the y-axis) as a function of the amount of sebum (on the x-axis). The skin hydration measurements and the amount of sebum are provided in arbitrary units (a.u.) in Fig. 8. The skin hydration measurements derived from the response measured using the device 100 comprising the spacer 104 are shown after correction for the contribution of the spacer 104. In this example, the spacer 104 comprises a polyethylene terephthalate (PET) foil with a thickness of 3 µm.

The skin hydration measurements are obtained from a calibration pad soaked with water and with different levels of sebum placed on the top surface of the pad. The sebum level is varied from 0-150 arbitrary units (a.u.) and this is measured with a Sebumeter. As can be seen from Fig. 8, as the sebum level is varied from 0-150 arbitrary units (a.u.), the use of the existing device without a spacer provides a 2.5 times reduction in the skin hydration measurements even though the baseline hydration levels are constant, whereas this factor is reduced to less than 30% using the device 100 comprising the spacer 104 described herein. Thus, as illustrated in Fig. 8, the influence of the amount of lipids on the determined hydration level can be minimized using the device 100 comprising the spacer 104 described herein.

There is thus provided herein an improved device 100 and method 300 of operating the device 100 for determining a hydration level of skin 200. There is also provided a computer program product comprising a non-transitory computer readable medium, the computer readable medium having a computer readable code embodied therein, the computer readable code being configured such that, on execution by a suitable computer or processor, the computer or processor is caused to perform the method described herein.

It will thus be appreciated that the embodiments described herein also apply to computer programs, particularly computer programs on or in a carrier, adapted to put the disclosure into practice. The program may be in the form of a source code, an object code, a code intermediate source and an object code such as in a partially compiled form, or in any other form suitable for use in the implementation of the method described herein. It will also be appreciated that such a program may have many different architectural designs. For example, a program code implementing the functionality of the method or device described herein may be sub-divided into one or more sub-routines. A variety of different ways of distributing the functionality among these sub-routines will be apparent to a person skilled in the art. The sub-routines may be stored together in one executable file to form a self-contained program. Such an executable file may comprise computer-executable instructions, for example, processor instructions and/or interpreter instructions (e.g. Java interpreter instructions).

Alternatively, one or more or all of the sub-routines may be stored in at least one external library file and linked with a main program either statically or dynamically, e.g. at run-time. The main program contains at least one call to at least one of the sub-routines. The sub-routines may also comprise function calls to each other. An embodiment relating to a computer program product comprises computer-executable instructions corresponding to each processing stage of at least one of the methods set forth herein. These instructions may be sub-divided into sub-routines and/or stored in one or more files that may be linked statically or dynamically. Another embodiment relating to a computer program product comprises computer-executable instructions corresponding to each means of the device set forth herein. These instructions may be sub-divided into sub-routines and/or stored in one or more files that may be linked statically or dynamically.

The carrier of a computer program may be any entity or device capable of carrying the program. For example, the carrier may include a data storage, such as a ROM, for example, a CD ROM or a semiconductor ROM, or a magnetic recording medium, for example, a hard disk. Furthermore, the carrier may be a transmissible carrier such as an electric or optical signal, which may be conveyed via electric or optical cable or by radio or other means. When the program is embodied in such a signal, the carrier may be constituted by such a cable or other device or means. Alternatively, the carrier may be an integrated circuit in which the program is embedded, the integrated circuit being adapted to perform, or used in the performance of, the method described herein.

Variations to the disclosed embodiments can be understood and effected by those skilled in the art in practicing the principles and techniques described herein, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored or distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. A device (100) for use in determining a hydration level of skin (200), the device (100) comprising:
at least two electrodes (102) configured to provide an electrical signal to the skin (200);
a spacer (104) arranged such that the at least two electrodes (102) provide the electrical signal to the skin (200) at different penetration depths (106, 108); and
a detector (110) configured to measure a response received from the skin (200) at the different penetration depths (106, 108) for use in determining the hydration level of the skin (200).

2. The device (100) as claimed in claim 1, wherein the penetration depths (106, 108) comprise any one or more of:
a penetration depth (106) from an electrode of the at least two electrodes (102) to a surface of the skin (200); and
a penetration depth (108) from an electrode of the at least two electrodes (102) to a location beneath the surface of the skin (200).

3. The device (100) as claimed in claim 1 or 2, wherein the spacer (104) comprises a dielectric spacer.

4. The device (100) as claimed in any of the preceding claims, wherein the spacer (104) is patterned such that the at least two electrodes provide electrical signal to the skin (200) at the different penetration depths (106, 108).

5. The device (100) as claimed in any of the preceding claims, wherein a composition and/or thickness of the spacer (104) is selected based on a geometry of the at least two electrodes (102).

6. The device (100) as claimed in any of the preceding claims, wherein the spacer (104) comprises a polyethylene terephthalate material, a polyelectric material, an aluminium material, or a polyetheretherketone material.

7. The device (100) as claimed in any of the preceding claims, wherein the spacer (104) is of a predefined thickness in a range from 1 micron to 100 microns.

8. The device (100) as claimed in any of the preceding claims, wherein the spacer (104) is arranged such that the spacer (104) is positioned between one or more of the at least two electrodes (102) and the skin (200) in use.

9. The device (100) as claimed in any of the preceding claims, wherein the at least two electrodes (102) comprise at least two pixelated electrodes.

10. The device (100) as claimed in any of the preceding claims, the device (100) further comprising:
a signal generator (112) configured to:
generate the electrical signal; and
provide the electrical signal to the at least two electrodes (102).

11. A system for determining a hydration level of skin (200), the system comprising:
a device (100) as claimed in any of the preceding claims; and
a processor (116) configured to:
acquire, from the detector (110), the measured response received from the skin (200) at the different penetration depths (106, 108); and
determine the hydration level of the skin (200) based on the measured response received from the skin (200) at the different penetration depths (106, 108).

12. A method of operating a device (100) for use in determining a hydration level of skin (200), the device (100) comprising at least two electrodes (102), a spacer (104), and a detector (110), wherein the method comprises:
providing, by the at least two electrodes (102), an electrical signal to the skin (200), wherein the spacer (104) is arranged such that the at least two electrodes (102) provide the electrical signal to the skin (200) at different penetration depths (106, 108); and
measuring, by the detector (110), a response received from the skin (200) at the different penetration depths (106, 108) for use in determining the hydration level of the skin (200).

13. The method as claimed in claim 12, the method further comprising:
generating, by a signal generator (112), the electrical signal; and
providing, by the signal generator (112), the electrical signal to the at least two electrodes (102).

14. The method as claimed in claim 12 or 13, wherein the method further comprises:
acquiring, by a processor (116) from the detector (110), the measured response received from the skin (200) at the different penetration depths (106, 108); and
determining, by the processor (116), the hydration level of the skin (200) based on the measured response received from the skin (200) at the different penetration depths (106, 108).

15. A computer program product comprising a non-transitory computer readable medium, the computer readable medium having a computer readable code embodied therein, the computer readable code being configured such that, on execution by a suitable computer or processor, the computer or processor is caused to perform the method as claimed in any of claims 12 to 14.
